# EUROPÄISCHE PATENTSCHRIFT

(11) **EP 0 503 229 B1**
(45) Veröffentlichungstag und Bekanntmachung des Hinweises auf die Patenterteilung: **21.06.1995**
(21) Anmeldenummer: 92100551.8
(22) Anmeldetag: 15.01.1992
(51) Int. Cl.: B01J 27/16, C07C 29/04, B01J 21/16

(54) **Verfahren zur Herstellung eines Katalysators für die Hydratation von Olefinen zu Alkoholen**
Method for production of catalyst for hydration of olefins to alcohols
Procédé de préparation d'un catalyseur pour l'hydratation d'oléfines en alcools

(30) Priorität: 13.03.1991 DE 4107973
(43) Veröffentlichungstag der Anmeldung: 16.09.1992
(73) Patentinhaber: HÜLS AKTIENGESELLSCHAFT, 45764 Marl (DE)
(72) Erfinder: Schlüter, Dietrich, Dr., W-6553 Sobernheim (DE); Baumeister, Franz-Josef, W-4350 Recklinghausen (DE); Schubert, Klaus-Peter, W-4690 Herne 2 (DE)

(56) Entgegenhaltungen:
- EP-A- 0 283 649
- GB-A- 1 075 895

## Beschreibung

Es ist bekannt, daß sich Olefine in der Gasphase bei erhöhten Drücken mit Wasserdampf zu Alkoholen umsetzen lassen. Besondere technische Bedeutung haben solche Verfahren bei der Herstellung von Ethanol aus Ethylen und Isopropylalkohol aus Propylen erlangt. Die Synthese dieser Alkohole wird in Gegenwart von Katalysatoren durchgeführt, und zwar dient in aller Regel als Katalysator Phosphorsäure, die auf Trägern aufgebracht ist.

Bekannt sind Trägermaterialien entweder auf der Basis reiner Kieselsäure, wie z. B. Kieselgur oder Kieselgel (US-PS 2 579 601), oder auf der Basis von Kieselsäure mit mehr oder weniger großem Tonerdegehalt, wie z. B. calcinierte Diatomeenerde, deren Struktur durch Ton oder tonhaltige Stoffe zusammengehalten wird (DE-PS 27 22 616, US-PS 3 311 568).

Bei den Trägern auf Basis reiner Kieselsäure ist die Festigkeit über längere Standzeiten problematisch. Die tonerdehaltigen Materialien zeichnen sich zwar durch eine bessere mechanische Festigkeit aus, sie haben jedoch bei zuhohem Tonerdegehalt den Nachteil, daß während der Reaktion das Aluminiumoxid durch die Einwirkung der Phosphorsäure herausgelöst wird.

In DE-PS 11 56 772 wurde nun ein Verfahren beschrieben, einen tonerdehaltigen Träger für die bei der Olefinhydratation als Katalysator verwendete Phosphorsäure herzustellen, indem geformte Kontaktkörper aus mineralischen Tonerdesilikaten derart mit Mineralsäure behandelt werden, daß der Aluminiumoxidgehalt vorzugsweise auf zwischen 1 und 5 Gewichtsprozent abgesenkt wird. Dieses Material weist im allgemeinen sowohl die erforderliche mechanische Festigkeit auf als auch einen ausreichend niedrigen Restaluminiumgehalt zur Vermeidung des Herauslösens. Andererseits wurde beim Einsatz von handelsüblichen Kontaktkörpern für die Herstellung von Katalysatorträgern für die Hydratation von Olefinen beobachtet, daß ohne Vorauswahl des Rohstoffs stark unterschiedliche Katalysatoraktivitäten gefunden werden.

Schließlich war es gelungen, auch auf der Basis grobporiger Kieselgele Träger für Phosphorsäure mit hoher Hydratationsaktivität und ausreichender mechanischer Festigkeit zu entwickeln (DE-PSS 25 25 705, 27 19 055). Allerdings verbleibt als Nachteil dieser Träger auf der Basis amorpher Kieselsäure, daß bei längerem Einsatz unter Bedingungen der Hydratationsreaktion die amorphe Kieselsäure teilweise kristallisiert, was mit starker Verminderung der spezifischen Oberfläche und damit der katalytischen Aktivität, und zwar irreversibel, verbunden ist, sowie mit einer Abnahme der mechanischen Festigkeit.

Ein weiterer Nachteil aller bisher eingesetzten Hydratationskatalysatoren aus Phosphorsäure auf silikatischem Träger ist das langsame Abnehmen der Aktivität durch ausgetragene Phosphorsäure, die im kontinuierlichen Betrieb ständig mit Lauge neutralisiert werden muß, um Korrosionswirkungen des sauren Rohalkohole auf nachgeschaltete Apparate zu vermeiden.

Durch kontinuierliche Einspritzung von Phosphorsäure gemäß DE-PS 26 58 946 in einer Menge, die der ausgetragenen Phosphorsäuremenge entspricht, konnte zwar der laufende Aktivitätsverlust weitgehend vermieden und damit die Katalysatorlebensdauer beträchtlich verlängert werden; damit sind aber entsprechende Anforderungen an die Lebensdauer des Trägers zu stellen, so daß die Verwendung solcher Träger ausscheidet, bei denen unter Reaktionsbedingungen Kristallisationen unter Verminderung der katalytischen Aktivität auf irreversible Weise ablaufen und die mechanische Festigkeit im Laufe der Zeit abnimmt.

Wie DE-PS 29 08 491 zeigt, läßt sich aus Tonmineralien dann ein Träger für einen Hydratationskatalysator gleichbleibend hoher katalytischer Aktivität erhalten, wenn durch sorgfältige Auswahl des Rohstoffs Vorsorge dafür getroffen wird, daß das Material in hohem Maße aus Montmorillonit besteht, was dazu führt, daß nach Formung, Auslaugung und Tränkung die Oberfläche und das Saugvolumen groß sind.

Die so hergestellten Katalysatoren bzw. Katalysatorträger aus montmorillonithaltigem Ton zeigen gegenüber solchen, hergestellt aus geformten Kontaktkörpern auf Basis mineralischer Tonerdesilikaten unterschiedlicher Provenienz, eine erhöhte Aktivität, d. h. es werden pro Stunde und 1 Katalysatorschüttung etwa 105 bis 110 g Ethanol bzw. ca. 300 g Isopropylalkohol gewonnen. Diese erhöhte Aktivität kann jedoch nur über einen längeren Zeitraum aufrechterhalten werden, wenn die ausgetragene Phosphorsäure, die bei Ethanol etwa 0,07 g pro Stunde und 1 Katalysatorschüttung beträgt, bei Isopropylalkohol etwa 0,01 g pro Stunde und 1 Katalysatorschüttung, durch kontinuierliche Zugabe der gleichen Säuremenge ausgeglichen wird. Diese ausgetragene Säure muß darüber hinaus noch mit Lauge neutralisiert werden. Die mechanische Festigkeit der Katalysatoren liegt in der Größenordnung von 70 bis 90 Newton/Kugel, was für das Beschicken der üblichen Reaktoren ausreichend ist.

Nachdem somit ein Katalysator mit ausreichender Lebensdauer und Langzeitfestigkeit gefunden war und auch die Phosphorsäureaustragung auf einen befriedigenden Wert zurückgedrängt war, blieben die überwiegend aus Silicagel bestehenden Träger lediglich hinsichtlich ihrer anfänglichen Aktivität den auf Basis von Montmorillonit hergestellten Trägern überlegen. So werden gemäß DE-PS 27 22 616 bis zu 144 g Ethanol pro Stunde und 1 Katalysatorschüttung erzeugt, allerdings nur 115 g Isopropylalkohol pro Stunde und 1 Katalysatorschüttung In US-PS 3 311 568 wird nun eine wesentlich höhere Katalysatoraktivität beschrieben, nämlich 240 g Ethanol pro Stunde und 1 Katalysatorausschüttung, wobei alle 24 Stunden Phosphorsäure zudosiert wird und insgesamt eine Beobachtung der Aktivität nur über 1 500 Stunden, also etwa 2 Monate, erfolgte. Es wird berichtet, daß die Festigkeit mit höherem Anteil von Bentonit steigt, beschrieben wird die Zumischung von 3 bis 5 % Bentonit zu der Diatomeenerde vor der Sinterung.

Schließlich wurde noch gemäß DE-OS 37 09 401 gefunden, daß die guten Katalysatoreigenschaften gemäß DE-PS 29 08 491 und DE-PS 29 29 919 hinsichtlich Langzeitfestigkeit und gutem Festhalten der Phosphorsäure erhalten bleiben und gleichzeitig die Aktivität, gemessen in erzeugter Alkoholmenge pro Zeiteinheit und Katalysatorvolumen, erheblich gesteigert werden kann, wobei diese Aktivität über einen Zeitraum von 6 Monaten praktisch unverändert bleibt, wenn man dem in der ersten Stufe mit Säure behandelten hoch montmorillonithaltigen Ton vor dem Formen zum Calcinieren 20 bis 40 Gewichtsprozent, bezogen auf die Gesamttrockensubstanz an einem feinkörnigen Silicagel zugibt und so die Oberfläche und Porenvolumen des fertigen Katalysatorträgers erhöht.

Da der Montmorillonit-Träger allein im fertig ausgelaugten Zustand vor der Tränkung mit Phosphorsäure nur einer Oberfläche von 150 bis 160 m²/g und ein Porenvolumen um 0,7 ml/g besitzt, ist unschwer zu erkennen, daß die Oberfläche durch den beispielsweise 30%igen Zusatz des Silicagels (Oberfläche ca. 350 m²/g) sich nur linear auf 180 bis 200 m²/g, dagegen das Porenvolumen (Zusatz: Silicagel 1,0 bis 1,2 ml/g) überproportional auf 0,95 bis 1,0 ml/g vergrößert, was ausschlaggebend für die Aktivitätssteigerung ist. Die Porendurchmesser des fertigen Trägers vor der Tränkung mit Phosphorsäure liegen zwischen 1 und 20 · 10⁻⁹ m, das Maximum der Häufigkeitsverteilung um 5 · 10⁻⁹ m.

Bei dem Verfahren gemaß der DE-PS 37 09 401 hat sich herausgestellt, daß eine Zunahme der Kugeldruckfestigkeit, die beim Naturprodukt Montmorillonit beim Nachcalcinieren auftritt, auch unter Prozeßbedingungen auf den Mischträger übertragen wird, während reines Silicagel unter Prozeßbedingungen immer an Festigkeit verliert.

Es war nun Aufgabe der Erfindung die Eigenschaften des Mischträgers weiter zu verbessern, insbesondere hinsichtlich der Festigkeit.

Überraschend wurde nun gefunden, daß durch Zusatz von feinkörnigem TiO₂ in bestimmten Mengen zu dem Montmorillonit-Silicagel-Träger eine Erhöhung der Kugeldruckfestigkeit eintritt.

Gegenstand der Erfindung ist daher ein Verfahren zur Herstellung eines Katalysators aus Tonmineralien und Silicagel für die Hydratation von Olefinen mit 2 bis 3 C-Atomen zu den entsprechenden Alkoholen mittels auf dem Trägermaterial aufgebrachter Phosphorsäure durch eine zweistufige Säurebehandlung dieses im wesentlichen montmorillonithaltigen Tons, der mit nicht mehr als 3 % Begleitmaterialien, wie Quarz, Feldspat und Glimmer, verunreinigt ist und bis zu 0,5 % K₂O enthalten kann und gegebenenfalls Formgebung und Calcinierung zwischen beiden Säurebehandlungen, wobei man dem in erster Stufe bis auf 10 bis 20 Gewichtsprozent, vorzugsweise 13 bis 18 Gewichtsprozent, Al₂O₃ säurebehandelten Ton, der eine Oberfläche von 200 bis 400 m²/g, bevorzugt 240 bis 300 m²/g, aufweist, 20 bis 40 Teile, bezogen auf die Gesamttrockensubstanz, feinkörniges Silicagel, zufügt, dann bei einem Gesamtwassergehalt von 40 bis 60 % durch Pressen formt, bei 500 bis 900 °C calciniert und anschließend das geformte Trägermaterial in einer zweiten Stufe mit Säure behandelt, bis es einen Al₂O₃-Gehalt von 1 bis 4 Gewichtsprozent, bevorzugt 1 bis 2 Gewichtsprozent, und eine Oberfläche von 180 bis 250 m²/g, bevorzugt 200 bis 220 m²/g, sowie ein Porenvolumen zwischen 0,8 und 1,5 ml/g, bevorzugt von 0,9 bis 1 ml/g, aufweist und den so erhaltenen Träger in bekannter Weise mit Phosphorsäure tränkt, wobei das zuzumischende feinkörnige Silicagel im wesentlichen eine Korngrößenverteilung zwischen 30 und 60 · 10⁻⁶ m, ein Porenvolumen von 0,9 bis 1,7 ml/g, vorzugsweise von 1 bis 1,2 ml/g, und eine Oberfläche von 200 bis 500 m²/g, bevorzugt 380 bis 400 m²/g, besitzt, das dadurch gekennzeichnet ist, daß man in der ersten Stufe vor Formgebung und Pressen noch zusätzlich zu dem Silicagel 1,5 bis 2,5 Teile Titandioxid, bezogen auf die Gesamttrockensubstanz, hinzufügt.

Die Zugabe von Titandioxid in Mengen von 1,5 bis 2,5 Teilen sollte nicht über- oder unterschritten werden. Optimal ist ein Mischträger aus 2 Teilen Titandioxid, 70 Teilen Bentonit und 30 Teilen Silicagel.

Hydrothermale Testversuche mit einem mit 40 % H₃PO₄ beladenen Träger mit 5 Teilen Titandioxid ergaben bei thermischer Belastung bei 250 °C und 70 bar und einem Stickstoff-Wasser-Verhältnis von 2,5 : 1 eine Verringerung des Porenvolumens (PV) des Trägers von 0,98 ml/g auf 0,75 ml/g bei überproportionaler Festigkeitszunahme von 80 Newton pro 150 Newton/Kugel. Eine Verringerung des PV führt immer zu Aktivitätsverlusten.

Zusatzmengen von nur etwa 1 Teil Titandioxid zeigten kaum eine Zunahme der Ausgangsfestigkeit.

Hydrothermale Testversuche mit einem Mischträger aus 2 Teilen Titandioxid, 30 Teilen Silicagel und 70 Teilen Bentonit zeigten kaum eine Veränderung des Gesamtporenvolumens, es blieb konstant, während die anfängliche Festigkeit von 80 Newton sich auf 120 Newton/Kugel steigerte.

Die Beschaffenheit des Titandioxids sollte in seiner Korngröße bevorzugt um 10⁻⁶ m liegen, um beim Vermischen der drei Komponenten eine gleichmäßige Verteilung im Endprodukt zu garantieren. Die Oberfläche sollte bevorzugt Werte in einem Bereich von 5-15 m²/g, besonders bevorzugt 10 m²/g, aufweisen und die analytische Reinheit sollte größer als 99,5 Gew.-%, cerimetrisch bestimmt, sein, um Nebenreaktionen auszuschließen.

### Beispiel 1

Ein gemahlener natürlicher Rohton, der aufgrund einer Laboruntersuchung so ausgewählt wurde, daß bei einer einstündigen Behandlung mit 20%iger Salzsäure bei 82 °C nicht mehr als 5 g K₂O pro kg eingesetzte Trockensubstanz extrahiert werden, wurde eine Stunde lang mit 20%iger Salzsäure auf 82 °C erhitzt, wobei die Säuremenge so bemessen wurde, daß auf 1 kg Ton 8,4 Mol HCl kamen, säurefrei gewaschen und getrocknet. Dabei wurde ein Material mit einem Restaluminiumgehalt von 16 Gewichtsprozent und einer spezifischen Oberfläche von 300 m²/g erhalten.

Zu 70 Teilen dieses getrockneten Materials wurden 30 Teile feinkörniges Silicagel der Fa. W. R. Grace, Worms, mit einer Korngrößenverteilung hauptsächlich zwischen 30 und 60 · 10⁻⁶ m, einem Porenvolumen von 1,1 ml/g und einer Oberfläche von 410 m²/g und 2 Teile Titandioxid mit einer Korngröße um 10⁻⁶ m und einer Oberfläche von 9 m²/g gegeben und nach dem Ansteigen bis zu einem Wassergehalt von 50 % zu Kugeln von 5 mm Durchmesser gepreßt. Die Verfestigung erfolgte durch 5 Stunden langes Erhitzen auf 800 °C.

Die so erhaltenen Kontaktkugeln wurden zweimal für je eine Stunde mit 20%iger Salzsäure bei 100 bis 110 °C behandelt und mit Wasser säurefrei gewaschen. Nach dem Trocknen bei etwa 110 bis 120 °C wurde bei den Kugeln ein Aluminiumoxidgehalt von 1,4 Gewichtsprozent und ein Titandioxidgehalt von 1,8 Gewichtsprozent ermittelt; die spezifische Oberfläche betrug 185 m²/g und das Porenvolumen 0,96 ml/g.

Die Kugeln wurden sodann mit 60 Gewichtsprozent Phosphorsäure getränkt und nach zweistündiger Einwirkzeit bei etwa 110 bis 120 °C getrocknet. Der H₃PO₄-Gehalt der so behandelten Kugeln betrug 45,1 Gewichtsprozent; die mittlere Druckfestigkeit lag bei 80 Newton/Kugel.

Beim Einsatz dieses so fertiggestellten Hydratationskatalysators zur Synthese von Ethanol aus Ethylen und Wasser in der Gasphase bei einer Temperatur von 235 °C und einem Druck von 70 bar konnte eine Katalysatorausbeute von 160 g Ethanol pro Stunde und 1 Katalysatorschüttung erhalten werden. Dieser Wert war nach sechsmonatiger Laufzeit der Anlage praktisch noch unverändert. Die Austragung an Phosphorsäure unter Reaktionsbedingungen belief sich auf 0,07 g pro Stunde und 1 Katalysatorschüttung.

Bei Ausbau des Mischträgers mit 2 Teilen Titandioxid wurde nun gefunden, daß die Kugeldruckfestigkeit auf 120 Newton/Kugel angestiegen war und selbst nach der Extraktion mit Wasser noch eine Festigkeit um 110 Newton/Kugel besaß. Das Porenvolumen war geringfügig auf 0,95 ml/g abgesunken, und die Oberfläche hatte sich auf ca. 30 m²/g reduziert. Parallel erfolgte eine Porengrößenverschiebung von Ø 10⁻⁸ m auf 10⁻⁶ m, was bei der entsprechenden Fahrzeit in der Synthese normal ist.

### Beispiel 2

Ein gemahlener natürlicher Rohton, der aufgrund einer Laboruntersuchung so ausgewählt wurde, daß bei einer einstündigen Behandlung mit 20%iger Salzsäure bei 82 °C nicht mehr als 5 g K₂O pro kg eingesetzte Trockensubstanz extrahiert werden, wurde eine Stunde lang mit 20%iger Salzsäure auf 82 °C erhitzt, wobei die Säuremenge so bemessen wurde, daß auf 1 kg Ton 8,4 Mol HCl kamen, säurefrei gewaschen und getrocknet. Dabei wurde ein Material mit einem Restaluminiumgehalt von 16 Gewichtsprozent und einer spezifischen Oberfläche von 300 m²/g erhalten.

Zu 70 Teilen dieses getrockneten Materials wurden 30 Teile feinkörniges Silicagel der Fa. W. R. Grace, Worms, mit einer Korngrößenverteilung hauptsächlich zwischen 30 und 60 · 10⁻⁶ m, einem Porenvolumen von 1,1 ml/g und einer Oberfläche von 410 m²/g gegeben und danach zu diesen 100 Teilen noch 2 Teile Titandioxid mit einer Korngrößenverteilung um 10⁻⁶ m und einer Oberfläche von 9 m²/g. Nach Ansteigen mit 50 % Wasser, auf die Gesamtmenge bezogen, wurde die Massen dann zu Kugeln von 5 mm Durchmesser gepreßt. Die Verfestigung erfolgte durch 5 Stunden langes Erhitzen auf 800 °C.

Die so erhaltenen Kontaktkugeln wurden zweimal für je eine Stunde mit 20%iger Salzsäure bei 100 bis 110 °C behandelt und mit Wasser säurefrei gewaschen. Nach dem Trocknen bei etwa 110 bis 120 °C wurde in den Kugeln ein Aluminiumoxidgehalt von 1,4 Gewichtsprozent und ein Titandioxidgehalt von 1,85 Gewichtsprozent gefunden; die spezifische Oberfläche betrug 200 m²/g, das Porenvolumen 0,94 ml/g.

Die Kugeln wurden sodann mit 40gewichtsprozentiger Phosphorsäure überflutet, die zwei Stunden einwirkte, danach wurde erneut bei etwa 110 bis 120 °C getrocknet. Die so behandelten Kugeln hatten einen H₃PO₄-Gehalt von 32 Gewichtsprozent. Die mittlere Druckfestigkeit betrug 80 Newton/Kugel.

Beim Einsatz dieses so fertiggestellten Hydratationskatalysators zur Synthese von Isopropylalkohol aus Propylen und Wasser in der Gasphase bei einer Temperatur von 186 °C und einem Druck von 38 bar konnte eine Katalysatorausbeute von 360 g Isopropylalkohol pro Stunde und 1 Katalysatorschüttung erhalten werden. Dieser Wert hatte sich nach sechsmonatiger Laufzeit der Anlage nicht verändert. Die Austragung an Phosphorsäure unter Reaktionsbedingungen betrug 0,005 g pro Stunde und 1 Katalysatorschüttung.

Auch hier stellte sich beim Ausbau des Mischträgers mit Titandioxid heraus, daß der Träger sich unter den zwar niedrigeren Temperaturbedingungen während des Hydratationsprozesses von 80 Newton auf 100 Newton/Kugel verfestigt hatte. Das Porenvolumen ist nach dem Entfernen der Phosphorsäure mit 0,93 ml/g praktisch konstant geblieben, während die Oberfläche auf etwa 40 m²/g abgefallen war, was sich aber beim Wiedertränken des Trägers mit Phosphorsäure auf die Aktivität beim erneuten Einsatz nicht negativ auswirkte.

### Beispiel 3 (Vergleich)

Ein gemahlener natürlicher Rohton, der aufgrund einer Laboruntersuchung so ausgewählt wurde, daß bei einer einstündigen Behandlung mit 20%iger Salzsäure bei 82 °C nicht mehr als 5 g K₂O pro kg eingesetzte Trockensubstanz extrahiert werden, wurde eine Stunde lang mit 20%iger Salzsäure auf 82 °C erhitzt, wobei die Säuremenge so bemessen wurde, daß auf 1 kg Ton 8,4 Mol HCl kamen, säurefrei gewaschen und getrocknet. Dabei wurde ein Material mit einem Restaluminiumgehalt von 16 Gewichtsprozent und einer spezifischen Oberfläche von 300 m²/g erhalten.

Zu 70 Teilen dieses getrockneten Materials wurden 30 Teile feinkörniges Silicagel der Fa. W. R. Grace, Worms, mit einer Korngrößenverteilung hauptsächlich zwischen 30 und 60 · 10⁻⁶ m, einem Porenvolumen von 1,1 ml/g und einer Oberfläche von 410 m²/g gegeben und nach dem Ansteigen bis zu einem Gesamtwassergehalt von 50 % zu Kugeln von 5 mm Durchmesser gepreßt. Die Verfestigung erfolgte durch 5 Stunden langes Erhitzen auf 800 °C.

Die so erhaltenen Kontaktkugeln wurden zweimal für je eine Stunde mit 20%iger Salzsäure bei 100 bis 110 °C behandelt und mit Wasser säurefrei gewaschen. Nach dem Trocknen bei etwa 110 bis 120 °C wurde in den Kugeln ein Aluminiumoxidgehalt von 1,5 Gewichtsprozent gefunden; die spezifische Oberfläche betrug 210 m²/g, das Porenvolumen 0,95 ml/g.

Die Kugeln wurden sodann mit 60gewichtsprozentiger Phosphorsäure überflutet, die zwei Stunden einwirkte, danach wurde erneut bei etwa 110 bis 120 °C getrocknet. Die so behandelten Kugeln hatten einen H₃PO₄-Gehalt von 44,8 Gewichtsprozent. Die mittlere Druckfestigkeit betrug 70 Newton/Kugel.

Beim Einsatz dieses so fertiggestellten Hydratationskatalysators zur Synthese von Ethanol aus Ethylen und Wasser in der Gasphase bei einer Temperatur von 235 °C und einem Druck von 70 bar konnte eine Katalysatorausbeute von 160 g Ethanol pro Stunde und 1 Katalysatorschüttung erhalten werden. Dieser Wert war nach sechsmonatiger Laufzeit der Anlage praktisch noch unverändert. Die Austragung an Phosphorsäure unter Reaktionsbedingungen betrug 0,07 g pro Stunde und 1 Katalysatorschüttung.

Beim Ausbau des Mischträgers wurde gefunden, daß die Kugeldruckfestigkeit unter Prozeßbedingungen von 70 Newton auf 90 Newton/Kugel angestiegen war, ein Merkmal, das wir bisher nur beim reinen Montmorillonit-Träger beobachtet hatten.

Bei einem Träger ohne Zusatz von Silicagel betrug die Oberfläche 160 m²/g vor der Tränkung mit Phosphorsäure, das Porenvolumen 0,7 ml/g. Unter gleichen Reaktionsgebedingungen, nämlich 70 bar und 235 °C wurde damit bei der Synthese von Ethanol aus Ethylen und Wasser pro Stunde und 1 Katalysatorschüttung eine Ethanolmenge von nur 135 g Ethanol erhalten.

## Patentansprüche

1. Verfahren zur Herstellung eines Katalysators aus Tonmineralien und Silicagel für die Hydratation von Olefinen mit 2 bis 3 C-Atomen zu den entsprechenden Alkoholen mittels auf dem Trägermaterial aufgebrachter Phosphorsäure durch eine zweistufige Säurebehandlung dieses im wesentlichen montmorillonithaltigen Tons, der mit nicht mehr als 3 % Begleitmaterialien, wie Quarz, Feldspat und Glimmer, verunreinigt ist und bis zu 0,5 % K₂O enthalten kann und gegebenenfalls Formgebung und Galcinierung zwischen beiden Säurebehandlungen, wobei man dem in erster Stufe bis auf 10 bis 20 Gewichtsprozent, vorzugsweise 13 bis 18 Gewichtsprozent, Al₂O₃ säurebehandelten Ton, der eine Oberfläche von 200 bis 400 m²/g, bevorzugt 240 bis 300 m²/g, aufweist, 20 bis 40 Teile, bezogen auf die Gesamttrockensubstanz, feinkörniges Silicagel, zufügt, dann bei einem Gesamtwassergehalt von 40 bis 60 % durch Pressen formt, bei 500 bis 900 °C calciniert und anschließend das geformte Trägermaterial in einer zweiten Stufe mit Säure behandelt, bis es einen Al₂O₃-Gehalt von 1 bis 4 Gewichtsprozent, bevorzugt 1 bis 2 Gewichtsprozent, und eine Oberfläche von 180 bis 250 m²/g, bevorzugt 200 bis 220 m²/g, sowie ein Porenvolumen zwischen 0,8 und 1,5 ml/g, bevorzugt von0,9 bis 1 ml/g, aufweist und den so erhaltenen Träger in bekannter Weise mit Phosphorsäure tränkt, wobei das zuzumischende feinkörnige Silicagel im wesentlichen eine Korngrößenverteilung zwischen 30 und 60 · 10⁻⁶ m, ein Porenvolumen von 0,9 bis 1,7 ml/g, vorzugsweise von 1 bis 1,2 ml/g, und eine Oberfläche von 200 bis 500 m²/g, bevorzugt 380 bis 400 m²/g, besitzt,
dadurch gekennzeichnet,
daß man in der ersten Stufe vor Formgebung und Pressen noch zusätzlich zu dem Silicagel 1,5 bis 2,5 Teile Titandioxid, bezogen auf die Gesamttrockensubstanz, hinzufügt.

2. Verfahren nach Anspruch 1,
dadurch gekennzeichnet,
daß der Mischträger aus 70 Teilen Bentonit, 30 Teilen Silicagel und 2 Teilen Titandioxid besteht.

## Claims

1. A process for the preparation of a catalyst from clay minerals and silica gel for the hydration of olefins having 2 to 3 C atoms to give the corresponding alcohols by means of phosphoric acid applied to the carrier, by two-stage acid treatment of this essentially montmorillonite-containing clay, which is contaminated with not more than 3% of accompanying materials, such as quartz, feldspar and mica, and may contain up to 0.5% of K₂O, and optionally shaping and calcination between both acid treatments, in which 20 to 40 parts, based on the total dry substance, of fine-particled silica gel are added in a first stage to the clay which is acid-treated to 10-20% by weight, preferably 13-18% by weight, of Al₂O₃ and has a specific surface area of 200 to 400 m²/g, preferably 240 to 300 m²/g, the mixture is then shaped at a total water content of 40 to 60% by compression and is calcined at 500 to 900°C, and the shaped carrier is then treated in a second stage with acid until it has an Al₂O₃ content of 1 to 4% by weight, preferably 1 to 2% by weight, and a specific surface area of 180 to 250 m²/g, preferably 200 to 220 m²/g, and a pore volume of between 0.8 and 1.5 ml/g, preferably of 0.9 to 1 ml/g, and the carrier thus obtained is impregnated with phosphoric acid in a known manner, the fine-particled silica gel to be admixed essentially having a particle size distribution of between 30 and 60 · 10⁻⁶ m, a pore volume of 0.9 to 1.7 ml/g, preferably 1 to 1.2 ml/g, and a specific surface area of 200 to 500 m²/g, preferably 380 to 400 m²/g, characterised in that, in the first stage, 1.5 to 2.5 parts, based on the total dry substance, of titanium dioxide are added in addition to the silica gel prior to shaping and compression.

2. A process according to claim 1, characterised in that the mixed carrier is composed of 70 parts of bentonite, 30 parts of silica gel and 2 parts of titanium dioxide.

## Revendications

1. Procédé pour fabriquer un catalyseur à partir de produits minéraux argileux et de gel de silice, pour l'hydratation d'oléfines ayant 2 ou 3 atomes de carbone en les alcools correspondants, à l'aide d'acide phosphorique appliqué sur le matériau support, par un traitement en deux étapes, par un acide, de cette argile contenant essentiellement de la montmorillonite, laquelle est contaminée au plus par 3 % d'impuretés telles que le quartz, le feldspath et le mica, et peut contenir jusqu'à 0,5 % de K₂O, et éventuellement un façonnage et une calcination entre les deux traitements par un acide, auquel cas on ajoute à l'argile traitée par un acide dans la première étape une quantité d'Al₂O₃ allant de 10 à 20 % en poids et de préférence de 13 à 18 % en poids, argile qui présente une aire de 200 à 400 m²/g et de préférence de 240 à 300 m²/g, 20 à 40 parties, par rapport à l'extrait sec total, d'un gel de silice à fine granulométrie ; puis on façonne par pressage en présence d'une teneur totale en eau de 40 à 60 % ; on calcine à une température de 500 à 900°C, puis le matériau support façonné est, dans une deuxième étape, traité avec un acide jusqu'à ce qu'il présente une teneur en Al₂O₃ de 1 à 4 % en poids et de préférence de 1 à 2 % en poids, et une aire de 180 à 250 m²/g et de préférence de 200 à 220 m²/g, ainsi qu'un volume de pores compris entre 0,8 à 1,5 ml/g et de préférence entre 0,9 et 1 ml/g ; et, d'une manière connue, on imprègne d'acide phosphorique le support ainsi obtenu, le gel de silice à fine granulométrie qu'il convient d'ajouter ayant essentiellement une répartition granulométrique comprise entre 30 et 60.10⁻⁶ m, un volume de pores de 0,9 à 1,7 ml/g et de préférence de 1 à 1,2 ml/g, et une aire de 200 à 500 m²/g et de préférence de 380 à 400 m²/g, caractérisé en ce que, dans la première étape, avant le façonnage et le pressage, on ajoute encore en plus au gel de silice 1,5 à 2,5 parties de dioxyde de titane par rapport à l'extrait sec total.

2. Procédé selon la revendication 1, caractérisé en ce que le support mixte est constitué de 70 parties de bentonite, de 30 parties de gel de silice et de 2 parties de dioxyde de titane.
